# EUROPEAN PATENT SPECIFICATION

(11) **EP 3 302 610 B1**
(45) Date of publication and mention of the grant of the patent: **19.01.2022**
(21) Application number: 16725648.6
(22) Date of filing: 29.04.2016
(51) Int. Cl.: A61M 1/16, B01J 20/06, B01J 20/34, B01J 20/02, B01J 20/28

(54) **ZIRCONIUM PHOSPHATE AND ZIRCONIUM OXIDE RECHARGING FLOW PATHS**
ZIRKONIUMPHOSPHAT- UND ZIRKONIUMOXIDWIEDERAUFLADEFLUSSWEGE
TRAJETS D'ÉCOULEMENT DE RECHARGE DE PHOSPHATE DE ZIRCONIUM ET D'OXYDE DE ZIRCONIUM

(30) Priority: 26.05.2015 US 201514722119; 26.05.2015 US 201514722068
(43) Date of publication of application: 11.04.2018
(73) Proprietor: Medtronic, Inc., Minneapolis, Minnesota 55432 (US)
(72) Inventor: MENON, Kanjimpuredathil, Muralikrishna, Bangalore Karnataka 560037 (IN); JEYACHANDRAN, Ramkumar, Bangalore Karnataka 560066 (IN); PATIL, Kaustubh, R., Bangalore Karnataka 560048 (IN); DUTTA, Sukalyan, Bangalore 560067 (IN); PRABHU, Gokul, L., Bangalore Karnataka 560016 (IN); LURA, David, B., Maple Grove, MN 55311 (US); PUDIL, Bryant, J., Plymouth, MN 55447 (US); HOBOT, Christopher, M., Rogers, MN 55374 (US)
(74) Representative: Maschio, Antonio
(86) International application number: PCT/US2016/030319
(87) International publication number: WO 2016/191041

(56) References cited:
- WO-A1-2015/060914
- WO-A1-2015/199765
- WO-A1-2015/199863
- WO-A1-2015/199864
- US-A1- 2015 251 161
- US-A1- 2015 251 162

## Description

### FIELD OF THE INVENTION

The invention relates to systems and methods for recharging zirconium phosphate and/or zirconium oxide used in sorbent dialysis. The systems and methods include rechargers, flow paths, related components, and control logic for simultaneously or independently recharging reusable modules containing zirconium phosphate or zirconium oxide.

### BACKGROUND

Zirconium phosphate and zirconium oxide are used in sorbent dialysis to remove waste and unwanted solutes from spent dialysate. Generally, zirconium phosphate removes ammonium, potassium, calcium, and magnesium ions from dialysate while the zirconium oxide removes anions such as phosphate or fluoride ions. Both materials are usually packaged together in a cartridge of some type or packed in separate cartridges. Usually, sorbent cartridges are discarded and replaced after use. The discarded sorbent cartridges are broken down and the individual materials separated from each other. Because zirconium phosphate and zirconium oxide are expensive and rechargeable, sorbent re-processers treat the recovered zirconium phosphate and zirconium oxide with a series of chemical solutions. The recycling process requires transporting the materials to reprocessing facilities and involves laborious recycling steps in addition to recharging the sorbent materials. Further, the sorbent material cannot be immediately reused, and must be added to a new sorbent cartridge and repackaged for sale. Safe disposal of the chemical waste from solutions used to recharge the materials may also require additional steps such as neutralizing the recharging solutions. Conventional methods drive up costs and infrastructure requirements, and increase complexity and waste.

Hence, there is a need for systems and methods that can quickly and effectively recharge sorbent materials without the need to remove the spent sorbent materials from the sorbent cartridge or sorbent modules. There is further a need for systems and methods that can quickly and effectively recharge different sorbent materials in a single recharging system. There is also a need for a system that can take advantage of the unique solutions necessary to recharge both zirconium oxide and zirconium phosphate to allow for automatic neutralization of the recharging solutions allowing safe disposal without additional treatment. There is further a need for systems and methods that can provide inline mixing of chemicals, reducing the volumes of chemicals needed.

WO 2015060914 provides a method for regenerating spent zirconium phosphate for reuse in sorbent dialysis treatments or other re-uses as a sorbent material. The method includes contacting spent zirconium phosphate with an aqueous disinfectant solution having at least one antimicrobial agent, and treating the resulting disinfected zirconium phosphate with an acidic solution to provide a treated zirconium phosphate that can be re-used as a sorbent material. Sorbent cartridge products containing the regenerated spent zirconium phosphate are also provided. Methods and systems for sorbent dialysis which re-use the regenerated zirconium phosphate such as part of sorbent cartridges, additionally are provided.

WO 2015199765 provides a method and apparatus for sorbent recharging. The method and related apparatus for recharging can recharge a specific rechargeable layer of a sorbent material such as zirconium phosphate in a sorbent cartridge. The method and apparatus include passing solutions containing combinations of acids, bases and salts through a module containing a rechargeable sorbent material such as zirconium phosphate in order to replace ions bound to the zirconium phosphate with hydrogen and sodium ions. The method allows for a customizable zirconium phosphate, with control over the ratios of sodium to hydrogen on the recharged zirconium phosphate.

WO 2015199863 and US 2015251161 each provides a method and related apparatus for sorbent recharging. The method and related apparatus for recharging can recharge a specific rechargeable layer of a sorbent material such as zirconium oxide and/or zirconium phosphate in a sorbent cartridge. The method and apparatus include passing solutions containing aqueous basic solutions through the zirconium oxide. The apparatus allows for recharging the zirconium oxide, and also for recharging zirconium phosphate, wherein the sorbent materials are contained in reusable sorbent modules.

WO 2015199864 and US 2015251162 provides a method and related apparatus for sorbent recharging. The method and related apparatus for recharging can recharge a specific rechargeable layer or module of a sorbent material such as zirconium phosphate in a sorbent cartridge. The method and apparatus include a fluid source containing at least one recharging fluid, wherein the fluid source is fluidly connectable to at least one rechargeable sorbent module for use in sorbent dialysis in a fluid flow path. The method and apparatus include passing a single solution through the zirconium phosphate for ion exchanges, resulting in zirconium phosphate to maintain a substantially consistent pH in a dialysate used during dialysis.

### SUMMARY OF THE INVENTION

The invention is defined by the appended claims.

### BRIEF DESCRIPTION OF THE DRAWINGS

FIG. 1A shows a recharging flow path for recharging zirconium phosphate and zirconium oxide.
FIG. 1B shows a recharging flow path for recharging zirconium phosphate and is an exploded left side of FIG. 1A.
FIG. 1C shows a recharging flow path for recharging zirconium oxide and is an exploded right side of FIG. 1A.
FIG. 2A shows a recharging flow path for recharging zirconium phosphate and zirconium oxide with inline mixing of recharging solutions.
FIG. 2B shows a recharging flow path for recharging zirconium phosphate with inline mixing of recharging solutions and is an exploded right side of FIG. 2A.
FIG. 2C shows a recharging flow path for recharging zirconium oxide with inline mixing of recharging solutions and is an exploded left side of FIG. 2A.
FIG. 3 shows a timeline for concurrent recharging of zirconium oxide and zirconium phosphate.
FIG. 4 shows material layers in a module sorbent cartridge including reusable modules.
FIG. 5 shows multiple sorbent modules connected together to form a sorbent cartridge.
FIG. 6 shows a recharger for recharging zirconium phosphate and zirconium oxide sorbent modules.
FIG. 7 shows a recharger fluidly connected to external fluid sources.
FIG. 8 shows multiple rechargers fluidly connected to a single set of fluid sources.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.*, to at least one) of the grammatical object of the article. By way of example, "an element" means one element or more than one element.

A "base source" is a fluid or concentrate source from which a basic solution can be obtained.

A "brine source" is a fluid or concentrate source from which a brine solution can be obtained. As used herein, a brine solution can refer to any solution comprising acids, bases and/or salts.

A "chamber" is a portion of a component or container physically separated from another portion of the component or container.

A "common reservoir" can be a container for collecting fluid of any type from one or more fluid sources including fluid lines or other reservoirs. The "common reservoir" can for example, store used or waste fluids.

The term "comprising" includes, but is not limited to, whatever follows the word "comprising." Use of the term indicates the listed elements are required or mandatory but that other elements are optional and may be present.

A "conductivity sensor" is a sensor configured to measure the conductivity of a fluid.

The term "consisting of' includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The terms "contain," "containing," or "contained" as used herein means to keep a material within a specific place. "Contain" can refer to materials placed within a component, absorbed onto a component, bound to a component, or any other method of keeping the material in a specific place.

The terms "conveying," "conveyed," or to "convey" refers to moving a fluid.

A "disinfectant source" is a fluid or concentrate source from which a disinfectant solution can be obtained. The disinfectant solution can be an acidic solution, such as a peracetic acid solution, or any other solution capable of disinfecting reusable sorbent modules.

The term "downstream" refers to a position of a first component in a flow path relative to a second component wherein fluid will pass by the second component prior to the first component during normal operation. The first component can be said to be "downstream" of the second component, while the second component is "upstream" of the first component.

A "drain" is a fluid line through which fluids may be disposed.

A "drain line" is a fluid line through which used or waste fluid may flow for disposal. The drain line can be connected to a drain, or to a container or reservoir for later disposal of the fluid.

An "effluent line" is a fluid passageway, tube, line, or path of any kind into which fluid exiting a container, module, or component will flow.

An "effluent line junction" is a location where at least two effluent lines are connected to each other, with or without a valve.

The term "external" refers to a component, module, or fluid line being situated or positioned outside of a casing or rigid structure.

A "flow sensor" is a device capable of measuring an amount or rate of fluid moving past or through a location.

A "fluid" is a liquid substance optionally having a combination of gas and liquid phases in the fluid. Notably, a liquid, as used herein, can therefore also have a mixture of gas and liquid phases of matter.

The term "fluidly connectable," "fluidly connect," "for fluid connection," and the like, refer to the ability of providing for the passage of fluid or gas or mixtures thereof, from one point to another point. The two points can be within or between any one or more of compartments, modules, systems, components, and rechargers, all of any type. The connection can optionally be disconnected and then reconnected.

A "fluid connector," "fluid connection," and the like describe a connection between two components wherein fluid, gas, or a combination thereof, can flow from one component, through a connector or a component for connection, to another component. The connector provides for a fluid connection in its broadest sense and can include any type of tubing, fluid or gas passageway, or conduit between any one or more components of the invention. The connection can optionally be disconnected and then reconnected.

The term "fluid line mixing" refers to mixing fluids at a location or junction wherein flow at the location of junction can, in part, mix one or more fluids.

A "heater" is a component capable of raising the temperature of a substance, container, or fluid.

A "heat exchanger" is a device comprising at least two chambers, wherein a fluid, gas, or combination thereof, can pass through one chamber, while a second fluid, gas, or combination thereof, can pass through the second chamber. Heat transfer occurs between the two chambers of the heat exchanger, such that if the fluids, gases, or combinations thereof, in opposite chambers are at different temperatures, the higher temperature fluid, gas, or combination thereof, will act to heat up the lower temperature fluid, gas, or combination thereof.

The term "mixing" generally refers to causing one or more fluids from any source to combine together. For example, "mixing" can include laminar or turbulent flow at a location in a fluid line or a junction. Another example of "mixing" can include receiving one or more fluids in a component configured to receive fluids from one or multiple sources and to mix the fluids together. Another aspect of mixing includes where one fluid is used to dissolve all or parts of a solid when the solid and fluid are brought together. Additionally, mixing can refer to the dissolution of a solid or solids with a fluid, wherein the solid or solids is dissolved in the fluid.

A "module inlet" is a connector through which a fluid, slurry, or aqueous solution can enter a sorbent module.

A "module outlet" is a connector through which a fluid, slurry, or aqueous solution can exit a sorbent module.

The term "positioned" or "position" refers to a physical location of a component or structure.

The term "pressure sensor" refers to a device for measuring the pressure of a gas or liquid in a vessel, container, or fluid line.

The term "pump" refers to any device that causes the movement of fluids, gases, or combinations thereof, by applying suction or pressure.

A "sorbent recharger", or generally referred to as a "recharger" herein, is an apparatus designed to recharge at least one sorbent material.

A "recharger housing" refers to a rigid casing or structure enclosing and protecting the components of the recharger flow paths and related components.

"Recharging" refers to treating a sorbent material to restore the functional capacity of the sorbent material to put the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged." Recharging of rechargeable sorbent materials is not the same as replenishing of a sorbent material such as urease. Notably, urease is not "recharged," but can be replenished, as defined herein.

A "recharging flow path" is a path through which fluid can travel while recharging sorbent material in a reusable sorbent module.

A "recharging solution" is a solution comprising the appropriate ions for recharging a specific sorbent material.

A "rinse loop" is a fluid pathway that connects one portion of a flow path to a second portion of a flow path and is designed to transfer fluids for rinsing a component.

The terms "sensing," "sensed," or to "sense" refer to determining one or more parameter or variable.

A "sensor" is a component capable of determining or sensing the states of one or more variables in a system.

A "sorbent cartridge module" or "sorbent module" means a discreet component of a sorbent cartridge. Multiple sorbent cartridge modules can be fitted together to form a sorbent cartridge of two, three, or more sorbent cartridge modules. In some embodiments, a single sorbent cartridge module can contain all of the necessary materials for dialysis. In such cases, the sorbent cartridge module can be a "sorbent cartridge."

A "static mixer" is a component configured to receive fluids from one or multiple sources and to mix the fluids together. The static mixer may include components that agitate the fluids to further mixing.

The term "upstream" refers to a position of a first component in a flow path relative to a second component wherein fluid will pass by the first component prior to the second component during normal operation. The first component can be said to be "upstream" of the second component, while the second component is "downstream" of the first component.

A "valve" is a device capable of directing the flow of fluid, gas, or combination thereof by opening, closing or obstructing one or more pathways to control whether the fluid, gas, or combination thereof, to travel in a path. One or more valves that accomplish a desired flow can be configured into a "valve assembly."

A "waste reservoir" is a container for collecting and storing used or waste fluids.

A "water source" is a fluid source from which water can be obtained.

A "zirconium oxide bypass line" refers to a fluid line that provides for movement of fluid between two points without passing through a zirconium oxide sorbent module.

A "zirconium oxide pump" is a pump positioned in a zirconium oxide recharging flow path

A "zirconium oxide recharging flow path" is a path through which fluid can travel while recharging zirconium oxide in a reusable zirconium oxide sorbent module.

A "zirconium phosphate bypass line" refers to a fluid line that provides for movement of fluid between two points without passing through a zirconium phosphate sorbent module.

A "zirconium phosphate pump" is a pump positioned in a zirconium phosphate recharging flow path.

A "zirconium phosphate recharging flow path" is a path through which fluid can travel while recharging zirconium phosphate in a reusable zirconium phosphate sorbent module.

### Flow Paths Recharging Sorbent Materials

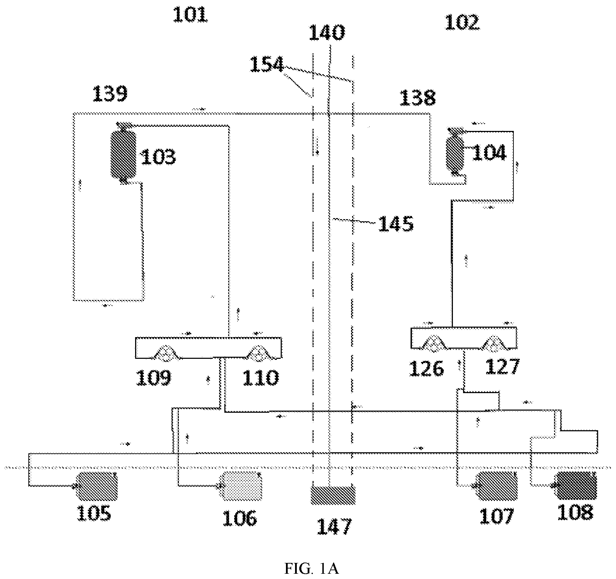

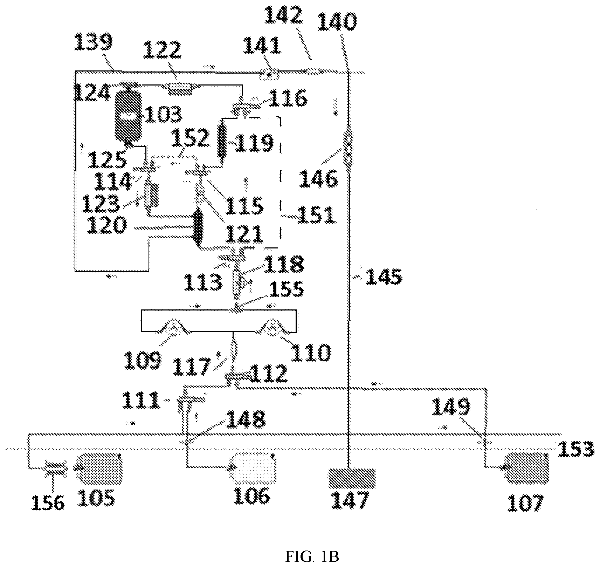

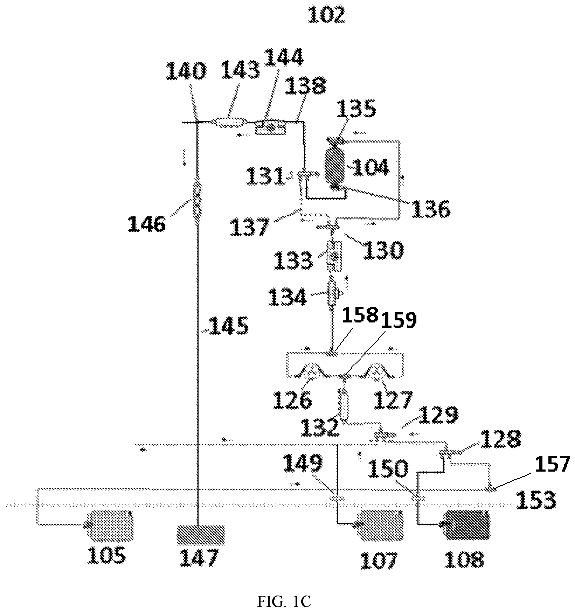

Flow paths can be used to recharge one or more reusable sorbent modules that separately contain zirconium phosphate or zirconium oxide. The flow paths described can also recharge multiple reusable sorbent modules and neutralize effluent waste solutions for safe and easy disposal. The flow paths of the invention can be arranged as shown in FIG.'s 1A-C. FIG. 1A is a generalized view of a recharging flow path, with details shown in FIG.'s 1B and 1C. The recharging flow path can be divided into a zirconium phosphate recharging flow path **101** containing the zirconium phosphate module **103** and a zirconium oxide recharging flow path **102** containing zirconium oxide module **104.** Details of the zirconium phosphate recharging flow path **101** on the zirconium phosphate side of line **154** are illustrated in FIG. 1B, while details of the zirconium oxide recharging flow path **102** on the zirconium oxide side of line **154** are illustrated in FIG. 1C. Although a dual cartridge recharger system is shown, single, two, three, or more multiple cartridge recharger systems are envisioned. Any one of the recharger cartridge systems can be linked together to share resources for recharging the sorbent cartridge and can be adapted for large scale use. Similarly, the linked rechargers can be scaled down as demand for recharging decreases. The modular recharging set-up having more or less rechargers based on demand can be advantageously used where required.

In FIG. 1A, a zirconium phosphate recharging flow path **101** has a water source **105**, a brine source **106**, a disinfectant source **107**, and a base source **108.** The brine source **106**, disinfectant source **107**, and/or base source **108** can be a column containing a dry bed of the brine, disinfectant, and/or base components. Alternatively, a powdered source of the brine, disinfectant, and/or base components can be used. The dry bed or powdered source can be dissolved with an aqueous solution. A static mixer (not shown) can mix the single line coming through the column prior to entering the zirconium phosphate module **103** or zirconium oxide module **104.** Recharging the zirconium phosphate in a zirconium phosphate module **103** requires water, brine, and disinfectant. The water source **105**, the brine source **106**, and the disinfectant source **107** are fluidly connected to the zirconium phosphate recharging flow path **101.** Similarly, recharging zirconium oxide module **104** in zirconium oxide recharging flow path **102** requires water, base, and disinfectant. The water source **105**, the disinfectant source **107**, and the base source **108** can be fluidly connected to the zirconium oxide recharging flow path **102.** The zirconium phosphate recharging flow path **101** and zirconium oxide recharging flow path **102** can be operated simultaneously or independently. Disinfectant source **107** can contain any type of disinfectant compatible with zirconium phosphate and zirconium oxide capable of disinfecting the reusable sorbent modules. In any embodiment, the disinfectant source **107** can contain peracetic acid. In any embodiment, the peracetic acid can be a solution of between 0.5 % and 2% peracetic acid in water. Alternatively, the disinfectant source **107** can contain any disinfectant compatible with zirconium phosphate and zirconium oxide, including bleach or citric acid. The brine source **106** can have an acid, a base, and a sodium salt.

During zirconium phosphate recharging, potassium, calcium, magnesium, and ammonium ions bound to the zirconium phosphate must be replaced by hydrogen and sodium ions. The final ratio of hydrogen to sodium ions on the recharged zirconium phosphate can be determined by the pH, buffer capacity, and sodium concentration of the brine solution used in the recharging process. As shown in FIG. 1B, the brine source **106** can be a mixture of sodium chloride, sodium acetate, and acetic acid. In one non-limiting brine solution, the sodium chloride concentration can be between 2.5M and 4.9M, the sodium acetate concentration can be between 0.3M and 1.1M, and acetic acid concentration can be between 0.2M and 0.8M. The water source **105** can contain any type of water, including deionized water. To recharge the zirconium phosphate in the zirconium phosphate module **103**, the disinfectant from disinfectant source **107** can flow to the zirconium phosphate module **103** to disinfect the zirconium phosphate module **103.** Fluid from the disinfectant source **107** can flow to valve **112** in the zirconium phosphate recharging flow path **101.** Zirconium phosphate pumps **109** and **110** provide a driving force to pump the fluid through the zirconium phosphate recharging flow path **101.** Use of two or more separate pumps can reduce wear on the pumps. Correspondingly, smaller pumps can be used. The two or more pumps can provide in-line mixing and intermittent pumping so at any given time, a single pump can pump fluid through the zirconium phosphate recharging flow path **101**. The two pumps can be used simultaneously or independently. The two or more pumps can provide fluid line mixing of one or more separate fluid streams when used simultaneously. The two or more pumps can operate asynchronously but used concurrently. For example, a first pump can operate for a time and a second pump remain off, then the first pump shut off with the second pump turning on. Multiple pumps at various timed pumping stages are envisioned as described herein. One of skill in the art will understand that a single zirconium phosphate pump can also accomplish the described pump functions.

Zirconium phosphate pumps **109** and **110** can pump fluid from disinfectant source **107** through valve **112** and valve **113** of FIG. 1B Fluid can be pumped through three-way junction **155** to valve **116** and into zirconium phosphate module **103** through zirconium phosphate module inlet **124.** The illustrated junctions combine the inlet chemicals or water pumped by the two pumps such that higher flow rates can be achieved. During filling, fluid inside zirconium phosphate module **103** can be forced through zirconium phosphate module outlet **125** and into zirconium phosphate module effluent line **139.** The disinfectant can be sequestered in the zirconium phosphate module **103** to ensure disinfection. Heater **119** upstream of the zirconium phosphate module **103** can heat the disinfectant because disinfection can become more efficient at elevated temperatures. After disinfection, zirconium phosphate module **103** can be rinsed using water from water source **105.** Zirconium phosphate pumps **109** and **110** can pump water from water source 105 through valves **111** and **112** to valve **113.** The water can then be pumped through valves **115** and **116** through the zirconium phosphate module **103** through zirconium phosphate module inlet **124**, out zirconium phosphate module outlet **125** and into zirconium phosphate module effluent line **139.** Water can be pumped through the zirconium phosphate module **103** until all of the disinfectant is removed.

Fluid from brine source **106** can be pumped through the zirconium phosphate module **103** to load the zirconium phosphate module **103** with the proper ratio of sodium and hydrogen ions. Zirconium phosphate pumps **109** and **110** can pump fluid from brine source **106** to valve **111.** The brine can follow the same pathway as the water through zirconium phosphate module **103** and into zirconium phosphate module effluent line **139.** Heater **119** upstream of the zirconium phosphate module **103** can heat brine because recharging can become more efficient at elevated temperatures. Heat exchanger **120** can lessen the load on heater **119.** One or more heat exchangers and one or more heaters can be used. The heat exchanger **120** can be fluidly connected to zirconium phosphate module effluent line **139** and to zirconium phosphate module inlet **124** upstream of heater **119**. The heated fluid exiting the zirconium phosphate module **103** in zirconium phosphate module effluent line **139** can heat the incoming brine solution in heat exchanger **120.** The heat exchanger **120** can have at least a first chamber and a second chamber. Fluid in the zirconium phosphate inlet lines can pass through the first chamber of the heat exchanger **120**, and fluid in the zirconium phosphate effluent line **139** can pass through the second chamber of the heat exchanger **120.** The increased temperature of the zirconium phosphate effluent in the second chamber can heat the fluid in the zirconium phosphate inlet lines in the first chamber. The zirconium phosphate module **103** can be rinsed again by pumping water through the zirconium phosphate module **103.** A static mixer (not shown) can be positioned upstream of the zirconium phosphate module **103** and mix the solutions prior to entering the zirconium phosphate module **103.**

Various sensors can be used in the zirconium phosphate module recharging flow path **101** to ensure proper concentrations and temperatures as shown in FIG. 1B. For example, conductivity sensor **117** can ensure that the incoming water contain no level of ions that may interfere with the recharging process, and that the brine solution and disinfectant solution are at a desired concentration. Conductivity sensor **142** can also be used to ensure that sufficient rinsing has occurred to remove brine and disinfectant solution. Pressure sensor **118** can monitor pressure in the zirconium phosphate inlet lines to ensure there are no occlusions or leaks and that the inlet pressures are in an acceptable range. Temperature sensor(s) **122** can ensure that the brine solution is at the proper temperature before entering zirconium phosphate module **103** and to control heater **119.** Temperature sensor **123** can be placed in zirconium phosphate module effluent line **139** to monitor the temperature of the effluent which can be controlled by heat exchanger **120** and heater **119.** A flow sensor **121** can monitor the flow rates of the fluids in the zirconium phosphate recharging flow path **101** and control zirconium phosphate pumps **109** and **110**. One of skill in the art will understand that alternative arrangements of sensors can be used in FIG. 1B and that one or more additional sensors can be added. Further, the sensors can be placed at any appropriate position in the zirconium phosphate recharging flow path **101** to determine fluid parameters at various locations throughout the zirconium phosphate recharging flow path **101**.

Zirconium phosphate bypass line **152** fluidly connects valve **115** to valve **114** in the zirconium phosphate effluent line **139**. Valves **115** and **116** can be controlled to direct fluid through the zirconium phosphate bypass line **152** and into zirconium phosphate effluent line **139**. The dual flow path aspect of the recharging flow path depicted in FIG. 1A can neutralize the effluent from both the zirconium phosphate module **103** and zirconium oxide module **104** by mixing the acidic effluent from the zirconium phosphate module **103** with the basic effluent from zirconium oxide module **104**. If only zirconium oxide module **104** is being recharged using the flow path of FIG. 1C, the zirconium phosphate bypass line **152** can be utilized to direct fluid from the brine source **106** to the zirconium phosphate effluent line **139** to neutralize the zirconium oxide effluent without the need to simultaneously recharge a zirconium phosphate module **103**. Similarly, bypass line **152** can directly connect the zirconium phosphate module inlet **124** to zirconium phosphate module outlet **125**. The zirconium phosphate recharging flow path **101** can include a rinse loop **151** to fluidly connect valve **113** upstream of the heater **119** and heat exchanger **120** to valve **116**, bypassing heater **119** and heat exchanger **120.** The rinse loop **151** can rinse brine solution from the zirconium phosphate module **103**. By bypassing heater **119** and heat exchanger **120** through rinse loop **151**, the zirconium phosphate module **103** can be cooled faster.

To recharge the zirconium oxide module **104** of FIG. 1C disinfectant from disinfectant source **107** can be first pumped to the zirconium oxide module **104** to disinfect the zirconium oxide module **104**. Fluid from the disinfectant source **107** can be pumped to valve **129** in the zirconium oxide recharging flow path **102**. Zirconium oxide pumps **126** and **127** can pump fluid through the zirconium oxide recharging flow path **102**. As described, a single zirconium oxide pump can be used as an alternative to the dual pump system in FIG. 1A. Also, two or more zirconium oxide pump are contemplated. The two or more zirconium oxide pumps can provide fluid line mixing of one or more separate fluid streams when used simultaneously. The two or more zirconium oxide pumps of FIG. 1C can be asynchronous but used concurrently. For example, a first pump can operate for a time and a second pump remain off, then the first pump shut off with the second pump turning on. Multiple pumps at various timed pumping stages are envisioned as described herein. Zirconium oxide pumps **126** and **127** pump fluid from disinfectant source **107** through valve **129** to valve **130**. The fluid flows to the zirconium oxide module **104** through zirconium oxide module inlet **135**. During filling, fluid inside zirconium oxide module **104** can flow through zirconium oxide module outlet **136** and into zirconium oxide module effluent line **138**. The disinfectant can be sequestered in zirconium oxide module **104** to ensure disinfection. The zirconium oxide module **104** can then be flushed with water from water source **105** after disinfection is completed. Zirconium oxide pumps **126** and **127** can pump water from water source **105** through valves **128** and **129** and junction **157** to valve **130**. The fluid passes through junctions **158** and **159** to reach valve **130**. The water can then be pumped to zirconium oxide module **104** through zirconium oxide module inlet **135** and out zirconium oxide module outlet **136** and into zirconium oxide module effluent line **138**. The zirconium oxide module **104** can be flushed with any volume of water required to ensure that the disinfectant is completely removed.

In FIG. 1C, zirconium oxide pumps **126** and **127** can pump fluid from base source **108** through valve **128** to zirconium oxide module **104**. The base source **108** can contain hydroxide ions to recharge zirconium oxide module **104**. The hydroxide ions can flow through zirconium oxide module **104** and into zirconium oxide module effluent line **138**. The base source **108** can be any suitable basic solution capable of replacing phosphate and other anions bound to the zirconium oxide with hydroxide ions. The hydroxide base can be any suitable base such as sodium hydroxide. One non-limiting example is sodium hydroxide having a concentration between 0.5M and 2.0M. Another non-limiting example is sodium hydroxide having a concentration at 90% or greater than 2% of the concentration of the recharging solution. A final rinse of the zirconium oxide module **104** can be performed by pumping water through the zirconium oxide recharging flow path **102** and zirconium oxide module **104**. Zirconium oxide recharging flow path **102** can also have a zirconium oxide bypass line **137** fluidly connecting valve **130** in the zirconium oxide inlet line to valve **131** in the zirconium oxide effluent line **138**. Valves **130** and **131** can direct fluid through the zirconium oxide bypass line **137** and into zirconium oxide effluent line **138**. Zirconium oxide bypass line **137** can convey fluid directly from the base source **108** to the zirconium oxide effluent line **138** to neutralize the zirconium phosphate effluent without the need to simultaneously recharge a zirconium oxide module **104**. Alternatively, zirconium oxide module inlet **135** can be fluidly connected to zirconium oxide module outlet **136.** Multiple sensors can be included in the zirconium oxide recharging flow path **102** to monitor fluid concentration. For example, conductivity sensor **132** can be used to monitor concentrations of the zirconium oxide recharging fluid; pressure sensor **134** can be used to monitor pressure in the zirconium oxide inlet line and to detect leaks or occlusions. Flow sensor **133** can determine the flow rate of the fluid through the zirconium oxide inlet line and be used to control zirconium oxide pumps **126** and **127**. A static mixer (not shown) can be positioned upstream of the zirconium oxide module **104** and mix solutions prior to entering the zirconium oxide module **104.** A heater and heat exchanger (not shown) can be positioned in the zirconium oxide recharging flow path **102** to heat fluids prior to entering zirconium oxide module **104**. Heating fluid in the zirconium oxide recharging flow path **102** can reduce recharging times and allow disinfection with a base solution, such as sodium hydroxide. Heating the fluid also allows for reduced disinfection time with a disinfectant source. A zirconium oxide rinse loop (not shown) can also be included to bypass the heater and heat exchanger during flushing.

Effluent from zirconium phosphate recharging flow path **101** can neutralize, either completely or in part, the effluent from zirconium oxide recharging flow path **102**, and vice versa. Zirconium phosphate effluent line **139** can be fluidly connected to zirconium oxide effluent line **138** at an effluent line junction **140** joining drain line **145**, which fluidly connects to drain **147**. Static mixer **146** can be used at or downstream of the effluent line junction **140** to mix zirconium phosphate effluent with zirconium oxide effluent.

Zirconium phosphate effluent line **139** and zirconium oxide effluent line **138** of FIG. 1A can be connected to a common reservoir for storage and disposal of the combined effluent. The common reservoir receives and collects the zirconium phosphate and zirconium oxide effluents together. The collected effluents can be drained after appropriate volumes of each effluent have been added to achieve neutralization. A common reservoir can allow for neutralization of the zirconium phosphate and zirconium oxide effluents without synchronizing the recharging processes. A single common reservoir could also be sized to support multiple rechargers.

Alternatively, the two fluid streams may be mixed through fluid line mixing at the effluent line junction **140**. Flow sensor **141** and conductivity sensor **142** can be placed in zirconium phosphate effluent line **139** to measure the flow rate and composition of the zirconium phosphate effluent. Flow sensor **144** and conductivity sensor **143** can be positioned in the zirconium oxide effluent line **138** to measure the wash and composition of the zirconium oxide effluent. Data from flow sensors **141** and **144** and conductivity sensors **142** and **143** can determine if the combined effluent in drain line **145** is safe for disposal into a drain. One non-limiting example of safe is an effluent having a pH in the range of 5.-9. Either zirconium phosphate effluent line **139** or zirconium oxide effluent line **138** can be connected simultaneously or independently to a waste reservoir (not shown) for disposal. Additional pH or conductivity sensors can be positioned downstream of the static mixer **146** to monitor and ensure safe disposal. Drain line **145** can also be connected to a common waste reservoir for storage and disposal of effluent. The common reservoir receives and collects the zirconium phosphate and zirconium oxide effluents together. The collected effluents can be drained after appropriate volumes of each effluent have been added to achieve neutralization. A common waste reservoir advantageously allows for neutralization of the zirconium phosphate and zirconium oxide effluents without synchronizing the recharging processes. Static mixer **146** may be unnecessary when a common reservoir is used.

Brine source **106**, disinfectant source **107**, and base source **108** can have filter **148**, filter **149**, and filter **150**, respectively to remove particulate matter. The one or more filters can remove particulate matter before fluid enters the zirconium oxide recharging flow path **102** or zirconium oxide recharging flow path **101.** Water source **105** can have microbial filter **156** to remove microbes from the water before entering the flow paths. In FIG. 1C, the dashed line **153** represents a recharger housing. The fluid sources can be external to the recharger housing and fluidly connected to the lines located inside of the recharger housing. Alternatively, the fluid sources described can instead be housed within the recharger.

During recharging, fluid can be passed through the zirconium phosphate module **103** and/or the zirconium oxide module **104** opposite to a flow direction used during dialysis. For example, zirconium phosphate module inlet **124** can be used as the zirconium phosphate module outlet during dialysis, and zirconium phosphate module outlet **125** can be the zirconium phosphate module inlet during dialysis in FIG. 1B. Similarly, zirconium oxide module inlet **135** can be used as the zirconium phosphate module outlet during dialysis, and zirconium oxide module outlet **136** can be used as the zirconium phosphate module inlet during dialysis. Pumping the recharging fluid through the modules in the opposite direction relative to dialysis can improve the efficiency of the recharging process.

The zirconium phosphate recharging flow path **101** or zirconium oxide recharging flow path **102** can independently recharge zirconium phosphate or zirconium oxide. For example, a single flow path fluidly connecting zirconium phosphate module **103** of FIG. 1B via valve **112** and valve **113** to each of the water source **105**, brine source **106**, and disinfectant source **107** can independently recharge the zirconium phosphate module **103.** Similarly, a single flow path fluidly connecting zirconium oxide module **104** of FIG. 1C via valve **128** and valve **129** to each of the water source **105**, disinfectant source **107**, and base source **108** can independently recharge the zirconium oxide module **104.**

The water source **105**, brine source **106**, disinfectant source **107**, and base source **108** can recharge one or more reusable sorbent module of various sizes. The amount of water, brine, disinfectant, and base depends on the concentration of each of the recharging solutions, the size of the reusable sorbent modules, the amount of cations/anions removed, and the flow rate used to pass the solutions through the reusable modules. The amount of brine solution required can depend on the temperature to which the brine solution is heated. For example, a brine solution having between 2.5 M and 4.9 M sodium chloride, between 0.3 M and 1.1 M sodium acetate, and between 0.2 M and 0.8 M acetic acid at between 70°C and 90°C requires between 4.2-6.2 L of brine to recharge a zirconium phosphate module containing between 2 kg and 3.2 kg of zirconium phosphate loaded with 2 to 3 moles of ammonium, calcium, magnesium and potassium. The brine solution should have a volume of at least between 4.2 and 6.2 L and delivered at a flow rate of between 100 and 300 mL/min. A single brine source can be connected to multiple rechargers, or can recharge multiple zirconium phosphate sorbent modules in a single recharger. The brine source can have a significantly larger volume from 1-100X or greater to ensure that the brine source need not be refilled each time a zirconium phosphate is recharged. For a zirconium oxide module having between 220 and 340 g of zirconium oxide loaded with 200 mmols of phosphate, a base source having between 0.5 and 2.0 M sodium hydroxide and a flow rate between 30 and 150 mL/min requires between 1 and 4.0 L of base. The base source can be at least between 1 and 4.0 L in volume. For recharging multiple zirconium oxide modules, a larger base source can be used.

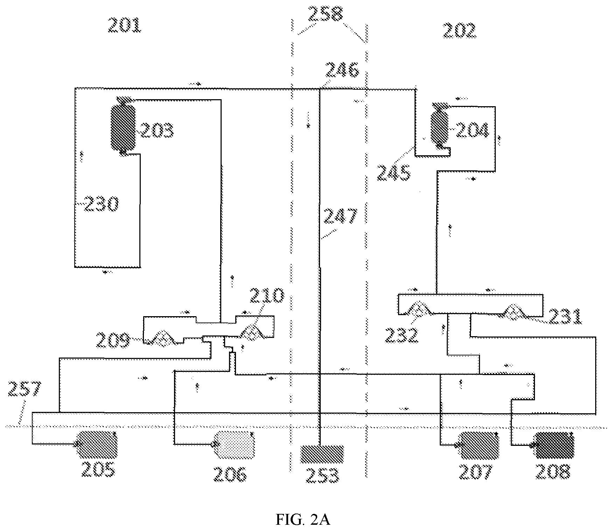

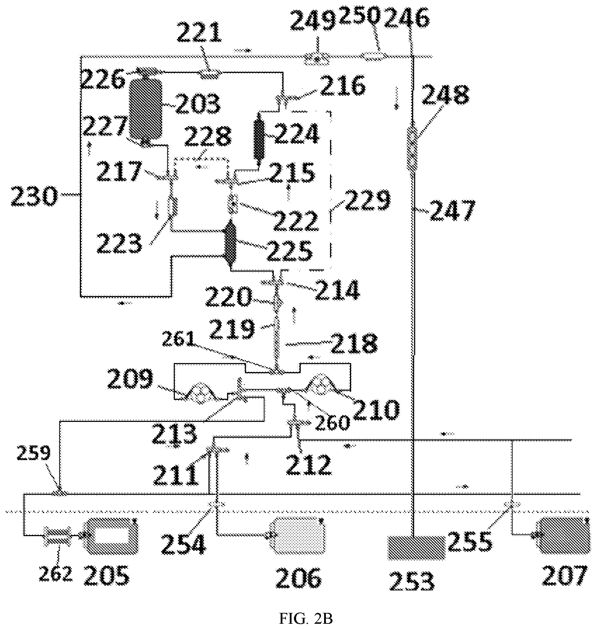

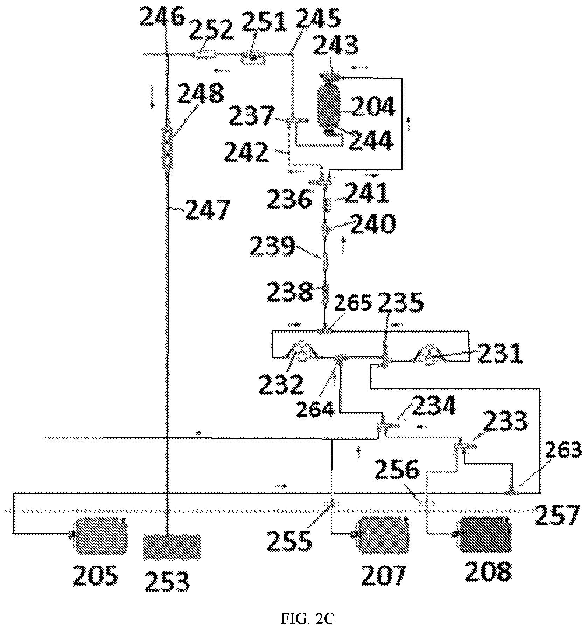

FIG. 2A is a generalized view of a recharging flow path having a zirconium phosphate recharging flow path **201** containing a zirconium phosphate module **203** and a zirconium oxide recharging flow path **202** containing a zirconium oxide module **204.** FIG. 2B illustrates a detailed view of zirconium phosphate recharging flow path **201** on the zirconium phosphate side of line **258**, and FIG. 2C illustrates a detailed view of zirconium oxide recharging flow path **202** on the zirconium oxide side of line **258.** The valves, pumps and static mixers illustrated in FIG.'s 2B and 2C allow for inline mixing of the recharging fluids. In FIG. 2A, the zirconium phosphate recharging flow path **201** and/or zirconium oxide recharging flow path **202** can be simultaneously or independently connected to a water source **205**, a brine source **206**, a disinfectant source **207**, and a base source **208**. Because recharging of the zirconium phosphate in a zirconium phosphate module **203** can require water, brine, and disinfectant, and because recharging of zirconium oxide in zirconium oxide module **204** can also require water, base, and disinfectant, the water source, **205**, the brine source **206**, and the disinfectant source **207** can be jointly connected to the zirconium phosphate recharging flow path **201**, and the water source **205**, the disinfectant source **207**, and the base source **208** can be jointly connected to the zirconium oxide recharging flow path **202.**

In FIG. 2A, zirconium phosphate recharging flow path **201** and zirconium oxide recharging flow path **202** can mix chemicals in-line to create the recharging solutions. Any one of disinfectant source **207**, brine source **206**, and base source **208** can contain solutions having concentrations over the concentration of the components to be used in recharging the reusable modules. Water source **205** can dilute the disinfectant, brine, and base from the fluid sources prior to recharging. In FIG. 2B, zirconium phosphate pump **210** can pump disinfectant into the zirconium phosphate module **203** with in-line mixing of concentrated disinfectant from disinfectant source **207** from valve **212** through junctions **260** and **261** and into static mixer **218**. Concurrently, zirconium phosphate pump **209** can pump water through junction **259** and valve **213** and into static mixer **218** from water source **205**. Alternatively, the concentrated disinfectant and water can be mixed through fluid line mixing at the junction of the two fluid lines. The zirconium phosphate pumps **209** and **210** can pump a disinfectant solution having a specified concentration and composition to disinfect the zirconium phosphate module **203** via valves **212** and **213**. The disinfectant solution can flow from static mixer **218** through valve **214** to valve **216** and then into the zirconium phosphate module **203** through zirconium phosphate module inlet **226**. Fluid can exit zirconium phosphate module **203** through zirconium phosphate module outlet **227** into zirconium phosphate effluent line **230**. After disinfection of zirconium oxide module **203**, zirconium phosphate pumps **209** and **210** can pump water from water source **205** into zirconium phosphate module **203**. For example, zirconium phosphate pump **209** can pump water through valve **213** to zirconium phosphate module **203** while zirconium phosphate pump **210** can pump water through valves **211** and **212** to zirconium phosphate module **203**. Alternatively, zirconium phosphate pump **209** can pump water through valves **211**, **212**, and **213** while zirconium phosphate pump **210** pumps water through valves **211** and **212**. During recharging, zirconium phosphate pumps **209** and **210** can pump brine through valve **211** to valve **212** from brine source **206** into static mixer **218**. If a concentrated brine solution is being used, zirconium phosphate pumps **209** and/or **210** can pump water from water source **205** to static mixer **218** to dilute the brine solution and generate a brine solution having a proper solute concentration for recharging the zirconium phosphate. After pumping brine through the zirconium phosphate module **203**, zirconium phosphate pump **209** can pump water through valves **211**, **212** and **213** while zirconium phosphate pump **210** can pump water through valve **211** and **212**.

The zirconium phosphate recharging flow path **201** of FIG. 2B can have a heater **224** and heat exchanger **225**. One or more heat exchangers and one or more heaters can be used. The brine solution can be heated by the heater **224** upstream of the zirconium phosphate module **203**. Heat exchanger **225** can utilize the heat from brine exiting the zirconium phosphate module **203** to heat the incoming brine solution upstream of heater **224** to reduce the burden on heater **224**. As described, the zirconium phosphate recharging flow path **201** can also have an optional zirconium phosphate bypass line **228** fluidly connecting valve **215** in the zirconium phosphate inlet line to valve **217** in the zirconium phosphate effluent line **230**. The zirconium phosphate bypass line **228** can neutralize the zirconium oxide effluent with brine even if the zirconium phosphate module **103** is not being recharged. Zirconium phosphate recharging flow path **201** can have a rinse loop **229** connecting valve **214** upstream of the heater **224** and heat exchanger **225** to valve **216** to bypass heater **224** and heat exchanger **225** to rinse brine out of the zirconium phosphate module **203**.

Various sensors can be included in the zirconium phosphate recharging flow path **201** to ensure fluid parameters are within acceptable ranges. In FIG. 2B, conductivity sensor **219** can be placed downstream of static mixer **218** to ensure mixing and specified recharging fluid concentrations. Pressure sensor **220** can measure the fluid pressure and to identify leaks or occlusions. Flow sensor **222** can determine the flow rate of the fluid entering the zirconium phosphate module **203** and be used to control zirconium phosphate pumps **209** and **210**. Temperature sensor **221** can determine if the recharging fluid is a proper temperature range upon entering zirconium phosphate module **203** and relay data to a processor (not shown) that can control heater **224**. Temperature sensor **223** can determine the temperature of the zirconium phosphate effluent prior to entering heat exchanger **225**. Other sensor arrangements, including any number of conductivity, pressure, flow, and temperature sensors can be used.

In FIG. 2C, zirconium oxide pump **232** can pump disinfectant from disinfectant source **207** through valve **234** and into static mixer **238** to disinfect the zirconium oxide module **204** in zirconium oxide recharging flow path **202**. Zirconium oxide pump **231** can pump water from water source **205** through valve **235** to static mixer **238** to dilute the disinfectant from disinfectant source **207** to provide in-line mixing of the disinfectant solution. The diluted disinfectant can then be pumped through valve **236** to zirconium oxide module inlet **243** and into zirconium oxide module **204**. Effluent from the zirconium oxide module **204** can exit through zirconium oxide module outlet **244** and into zirconium oxide effluent line **245**. After disinfection, the disinfectant can be rinsed from the zirconium oxide module **204** by pumping water from water source **205** through valve **235** to zirconium oxide module **204** by zirconium oxide pump **231** while zirconium oxide pump **232** pumps water through valves **233** and **234** to zirconium oxide module **204**. Alternatively, zirconium oxide pump **231** can pump water through valves **233**, **234**, and **231**, while zirconium oxide pump **232** pumps water through valves **233** and **234**. To recharge zirconium oxide module **204**, zirconium oxide pump **232** can pump base from base source **208** through valves **233** and **234** through junctions **264** and **265** to static mixer **238**. Water from water source **205** can be pumped by zirconium oxide pump **231** through junctions **263** and **265** into static mixer **238** to dilute the base by in-line mixing. Alternatively, the water and base can be mixed through fluid line mixing at the junction of the two fluid lines. Alternatively, the base can be pre-set using specified amounts of base in pre-packaged packets or containers. Diluted base can flow through the zirconium oxide recharging flow path **202** and through zirconium oxide module **204**. The zirconium oxide module **204** can be rinsed any numbers of times, as needed, by introducing water from water source **205** to the zirconium oxide module **204**. The zirconium oxide recharging flow path **202** can also have a zirconium oxide bypass line **242** that fluidly connects valve **236** to valve **237** in the zirconium oxide effluent line **245** to bypass zirconium oxide module **204**. In this way, zirconium phosphate effluent can be neutralized with a base solution even if the zirconium oxide module **204** is not being recharged. A heater and heat exchanger (not shown) can be positioned in the zirconium oxide recharging flow path **202** to heat fluids prior to entering zirconium oxide module **204**. A zirconium oxide rinse loop (not shown) can also be included to bypass the heater and heat exchanger. Similarly, the zirconium oxide recharging flow path **202** can also have sensors for measurement and control over the recharging process. In FIG. 2C, a conductivity sensor **239** can be placed downstream of static mixer **238** to ensure that diluted recharging solutions have a desired concentration. Pressure sensor **240** can detect the pressure in the zirconium oxide recharging flow path **202** to detect leaks or occlusions. Flow sensor **241** can detect the flow rate of fluid in the zirconium oxide recharging flow path **202** and can be used to control zirconium oxide pumps **231** and **232**.

As shown in FIG. 2A, the present invention can provide in-line neutralization of the effluent from each of the zirconium phosphate recharging flow path **201** and zirconium oxide recharging flow path **202**. The zirconium phosphate effluent line **230** can be fluidly connected to zirconium oxide effluent line **245** at effluent line junction **246** and fluidly connected to drain line **247**. As shown in FIG.'s 2B and 2C, a static mixer **248** can be positioned at or downstream of the effluent line junction **246** to ensure mixing of the effluents from the zirconium phosphate recharging flow path **201** and zirconium oxide recharging flow path **202**. The combined effluent can be conveyed through the drain line **247** to drain **253**, or to a common waste reservoir (not shown), or to separate waste reservoirs. A conductivity sensor **250** as shown in FIG. 2B in zirconium phosphate effluent line **230** and a conductivity sensor **252** as shown in FIG. 2C in zirconium oxide effluent line **245** can determine the composition of the effluents. Flow sensor **249** in zirconium phosphate effluent line **230** of FIG. 2B and flow sensor **251** in zirconium oxide effluent line **245** of FIG. 2C can be used simultaneously or independently to measure the flow rates of each of the effluents. Determining the composition of the effluent fluids as well as the respective flow rates using one or more sensors described can monitor the system function and ensure that the combined effluent in drain line **247** is safe for disposal or storage.

Brine source **206**, disinfectant source **207**, and base source **208** can have filter **254**, filter **255**, and filter **256**, respectively to remove particulate matter prior to entering zirconium phosphate recharging flow path **201** or zirconium oxide recharging flow path **202**. The filters can also act as inline mixers to mix the solutions. Water source **205** can have microbial filter **262** to remove microbes from the water. Brine source **206**, disinfectant source **207**, and base source **208** can be housed outside of a recharger housing denoted by line **257**. The brine solution, disinfectant solution, and base solution can be generated through in-line mixing as described. Alternatively, pre-mixed solutions, concentrates, or infusates can be introduced into brine source **206**, disinfectant source **207**, and base source **208** and delivered to zirconium phosphate recharging flow path **201** or zirconium oxide recharging flow path **202**. For example, the brine solution in brine source **206** can be pre-mixed or provide in pre-packaged amounts in the proper concentrations and introduced into brine source **206**, disinfectant source **207**, and base source **208**.

In-line mixing can provide higher concentrations of solutes, lower fluid volumes required by the system, and physically smaller fluid reservoirs. The fluids should have suitable concentrations for use in the zirconium phosphate recharging flow path **201** or zirconium oxide recharging flow path **202**. An initially high source of disinfectant, such as peracetic acid, can be used in a concentration of between 20% and 40%. The zirconium phosphate recharging flow path **201** of FIG. 2B can dilute the peracetic acid or other disinfectant source by a factor of 20:1 to 40:1 to generate an acidic recharging solution having a concentration between 0.5 % and 2%. The initial disinfectant concentration can be any concentration greater than 1%. Similarly, the base solution can be sodium hydroxide having an initial concentration between 14M and 22M. The zirconium oxide recharging flow path **202** of FIG. 2C can dilute the base solution by 18:1 to 22:1 to generate a base solution having a concentration between 0.8 and 1.0 M. The initial base solution concentration can be any concentration greater than or equal to 0.5 M. The brine solution can also be diluted in-line to generate a brine solution having a proper recharging concentration. One example is a brine concentrate at 4.90M NaCl, 0.40M sodium acetate and 0.26M acetic acid diluted to 3.7M NaCl, 0.30M sodium acetate, and 0.20M acetic acid. The brine source **206** of FIG. 2A can be one or more reservoirs. For example, an acetic acid source, a sodium acetate source and a sodium chloride source can each be connected in place of single brine source **206**. Alternatively, an acetic acid source, a base source, and a sodium chloride source can be connected in place of the single brine source **206** with mixing of the base and acetic acid to generate the sodium acetate. The individual components can be added to the zirconium phosphate recharging flow path **201** in the proper ratios to generate the recharging brine.

The chemicals used in the recharging process can be packaged and shipped in any form. The chemicals can be packaged and shipped as solutions, either in proper concentrations for recharging or with higher concentrations for inline mixing. In any embodiment, the chemicals may be packaged and shipped in pure form, such as 100% acetic acid or solid sodium chloride, sodium acetate, or sodium hydroxide.

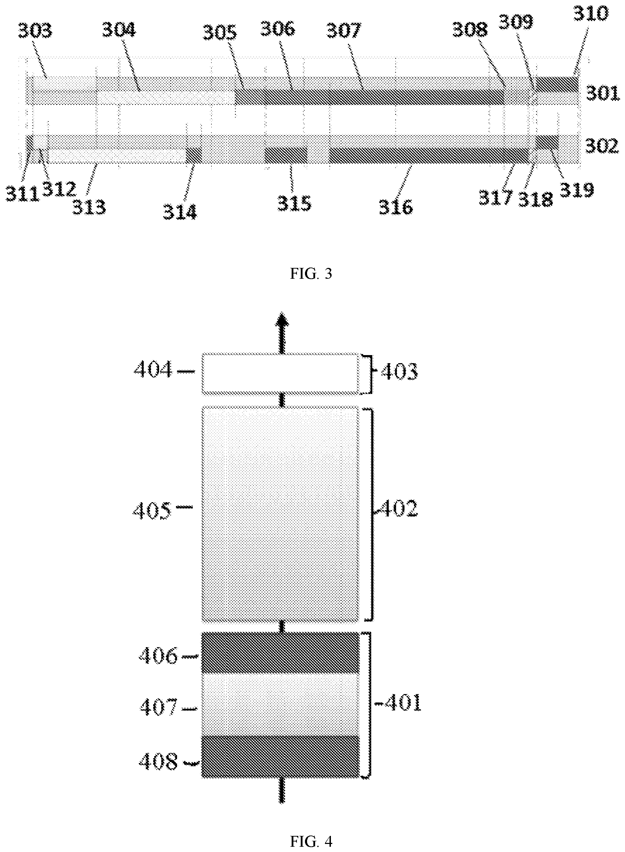

FIG. 3 illustrates a non-limiting example of a timeline that can be used for concurrent or separate recharging of zirconium phosphate and zirconium oxide. Timeline **301** shows recharging zirconium phosphate and timeline **302** shows recharging zirconium oxide. As illustrated in timeline **301**, the zirconium phosphate recharging process can begin by introducing a disinfectant, such as peracetic acid, into the zirconium phosphate module, shown as step **303**. The time necessary to fill the zirconium phosphate module with the disinfectant can depend on the flow rate of the disinfectant solution and the volume of the zirconium phosphate module. The disinfectant can be delivered to the zirconium phosphate module in step **303** at a flow rate of between 100 and 500 mL/min, which can fill a zirconium phosphate module in a time of between 5-10 minutes. Longer or shorter flushing times can be used depending on the need. After filling the zirconium phosphate with the disinfectant solution, the disinfectant solution can be held in the zirconium phosphate module to ensure disinfecting of the zirconium phosphate module in step **304**. In any embodiment, the disinfectant can be held in the zirconium phosphate module for any length of time sufficient to disinfect the zirconium phosphate module, including between 5 and 20 minutes. The temperature of the disinfectant can be determined with a temperature sensor, and the hold time adjusted as necessary. For example, if the disinfectant temperature is 22°C, the hold time can be 5 minutes. The disinfectant can also be heated to minimize the necessary hold time by heating the disinfectant to room temperature if necessary. During the hold time, the disinfectant flow can be stopped or reduced to a low flow condition, such as 5 to 75 ml/min. Holding the disinfectant in the module can build up pressure in the module, requiring periodic venting. To maintain the volume after venting, during which some fluid may leak, the disinfectant can be pumped into the module at a low flow rate during the venting. Alternatively, during the hold time, the disinfectant flow rate can be set to between 5 and 75 ml/min to prevent pressure buildup while maintaining fluid volume in the modules. The disinfectant solution can then be flushed from the zirconium phosphate module in step **305** by pumping water through the zirconium phosphate module. The water can flow through the zirconium phosphate module at a specified rate. A higher flow rate of the water in step **305** will cause a quicker flush time. The water can be pumped through the zirconium phosphate module at a rate of between 300 and 500 mL/min. Depending on the size of the zirconium phosphate module, the zirconium phosphate module can be flushed in about 5-10 minutes. As described, the system can utilize one or more sensors, such as pH sensors or conductivity sensors in the zirconium phosphate effluent lines to determine if disinfectant is fully flushed in step **305**. After flushing the disinfectant from the zirconium phosphate module in step **305**, brine solution can be pumped through the zirconium phosphate module to recharge the zirconium phosphate module starting in step **306**. The brine solution can be pumped through the zirconium phosphate module in step **306** at any rate. One of skill in the art will understand that a higher flow rate of brine solution may decrease the time necessary to recharge the zirconium phosphate, but may also decrease the efficiency of the process, resulting in the need for additional brine. Conductivity or pH sensors can be used to determine if the zirconium phosphate module has been fully filled with brine.

The brine flow rate can be set to any flow rate, including between 150 and 250 mL/min. Depending on the size of the zirconium phosphate module, between 5 and 10 minutes may be needed for brine to reach the sensors in the zirconium phosphate effluent line. Once brine has reached the sensors in the effluent line, the brine can flow through the zirconium phosphate module in step 307 until recharging is complete. Recharging time can vary based on the flow rate of the brine solution, the concentration of the brine solution, and the temperature of the brine solution. For example, the brine solution can be heated during the recharging process between 65°C and 95°C. Recharging of zirconium phosphate can be more efficient at elevated temperatures. Conductivity sensors can determine if step **308** has been completed by detecting the conducting of the fluid in the zirconium phosphate effluent line. If the conductivity of the effluent matches the conductivity of the brine, then no additional ions from the brine are being exchanged onto the zirconium phosphate, and recharging is complete. For example, steps **308**, **309**, and **310** represent brine solution being flushed from the zirconium phosphate module with water. Flushing can continue through step **310** until the conductivity sensors in the zirconium phosphate effluent line determine no additional brine is being removed from the zirconium phosphate module.

As depicted in timeline **302**, zirconium oxide can be recharged concurrently or independently of zirconium phosphate. In step **311**, zirconium oxide recharging begins by rinsing the zirconium oxide module with water. The water rinse can flush leftover dialysate bicarbonate or any sodium hydroxide from the flow loop, which may react violently with acid necessary for disinfection. After flushing the zirconium oxide module with water in step **311**, disinfectant solution can be delivered to disinfect the module in step **312**. The time necessary to fill the zirconium oxide module with disinfectant depends on the size of the zirconium oxide module and the flow rate of the disinfectant. Because less zirconium oxide is needed for dialysis than zirconium phosphate, the zirconium oxide module may be smaller than the zirconium phosphate module, and therefore fill faster in step **312** as compared to the zirconium phosphate module in step **303**. Upon filling, the disinfectant can be sequestered in the zirconium oxide module to allow for disinfection in step **313**. The disinfectant can be held in the zirconium oxide module for any length of time, including between 5and 20 minutes. The temperature of the disinfectant can be determined with a temperature sensor, and a hold time adjusted as necessary. For example, if the disinfectant temperature is 22°C, the hold time can be 5 minutes. The disinfectant can also be heated to minimize the necessary hold time. Upon disinfection, the disinfectant can be flushed from the zirconium oxide module in step **314**.

In step **315** the base solution flows through the zirconium oxide module to recharge the zirconium oxide. Step **315** continues until a basic solution is detected in the zirconium oxide effluent line. During simultaneous recharging, the basic effluent from the zirconium oxide recharging flow path neutralizes the acidic effluent from the zirconium phosphate recharging flow path. Once a basic effluent is detected in step **315**, the zirconium oxide recharging process can be halted until the acid brine is detected in the effluent of the zirconium phosphate module in step **306**, which may occur later due to size differences of the zirconium phosphate and zirconium oxide modules. After the acidic effluent is detected in the zirconium phosphate module, shown as step **306**, the base can continue to flow through the zirconium oxide module in step **316**. The flow rate of the base solution in step **316** can be any suitable rate. For example, the flow rate of the base solution can be between 30 and 150 mL/min. To ensure neutralization, the flow rate of the base in step **316** can depend on the flow rate of the brine in step **307**. As described, the base and effluent are each brought to a point equidistant to a junction between the zirconium phosphate and zirconium oxide effluent lines. Based on the conductivity of each effluent, the pumping is restarted at a ratio of speed that is needed for neutralization. The ratio could be 1:1 or any other ratio. Although described as using a conductivity sensor, the system can alternatively use a pH sensor or a combination of pH and conductivity sensors. A neutralization ratio can be calculated based on the relative pH, buffer capacity, and concentration of the zirconium phosphate effluent and zirconium oxide effluent. For example, a neutralization ratio of 1.5:1 means that 1.5 liters of the zirconium phosphate effluent will be required to fully neutralize one liter of zirconium oxide effluent. The flow rate of the base in step **316** can be set to half the flow rate of the brine solution, allowing full neutralization of both solutions. For example, the flow rate of the base in step **316** can be between 75 and 125 mL/min if the neutralization ratio is 1.5:1 and the brine flow rate is between 150 and 250 mL/min.

After the brine solution is detected in the effluent of the zirconium phosphate and the flushing of the brine begins in step **308**, the base solution can pass through the zirconium oxide module, shown as step **317** until the brine is mostly or fully flushed from the zirconium phosphate module, shown as step **309**. At this point, the base solution can be flushed from the zirconium oxide module, shown as step **318**. After confirming that the base has been flushed from the zirconium oxide module, flushing is completed in step **319**.

One of skill in the art will understand that the times and flow rates described in FIG. 3 can be altered within the scope of the invention. Higher flow rates can cause faster recharging of the modules. Times can be decreased by using more concentrated solutions, but may decrease efficiency. Specified concentrations, flow rates, and times can be set per the needs of the user, taking into account the cost of chemicals and need for fast recharging. The times and flow rates shown in zirconium oxide recharging timeline **302** can also be altered to reduce idle time. For example, the flow rate of the base solution in step **315** can be slowed down to reduce the time gap between steps **315** and **316**. If a single sorbent module is being recharged independently, or if a common waste reservoir is used for the zirconium phosphate and zirconium oxide recharging flow paths either inside or outside of the recharger,, the times and flow rates shown in FIG. 3 can be adjusted. Synchronizing the zirconium phosphate timeline **301** with the zirconium oxide timeline **302** is unnecessary because effluent is no longer neutralized in-line.

The zirconium oxide and zirconium phosphate sorbent modules can be recharged and reused any number of times. Alternatively, the sorbent modules may have a defined useful life, including a maximum number of recharge and reuse cycles. When a sorbent module reaches the end of the sorbent module's useful life, the sorbent module can be recycled or disposed of. A disinfection only cycle can disinfect the sorbent modules for safe disposal and/or recycling at the end of the sorbent module's useful life. In a disinfection only cycle, the disinfectant can be pumped into the sorbent module as described but the other recharge solutions would not be used. After disinfection, and optionally rinsing of the sorbent module, the sorbent module can be disposed or recycled safely.

A non-limiting embodiment of a reusable sorbent cartridge having modules that can be separated and recharged by systems and methods of the present invention is in FIG. 4. The sorbent cartridge can be separated into reusable modules to facilitate recharging of one or more sorbent materials. In FIG. 4, the sorbent cartridge has a first sorbent module **401**, a second sorbent module **402**, and a third sorbent module **403**. The first module **401** can have a layer of activated carbon **408**, a layer of alumina and urease **407**, and a second layer of activated carbon **406**. The activated carbon can remove many non-ionic solutes from the dialysate. The urease catalyzes the conversion of urea in the dialysate into ammonium ions. The alumina can serve as a support for the urease. The second layer of activated carbon **406** can capture any urease that migrates out of alumina and urease layer **407** prior to exiting the first module **401**. The first module **401** can be a single use module, or can be a multiple use module with replenishment of the urease. The second module **402** can have zirconium phosphate **405**. After dialysis, zirconium phosphate **405** will contain bound potassium, calcium, magnesium, and ammonium ions, which can be replaced with sodium and hydrogen ions by the recharging process described herein. Third module **403** can contain zirconium oxide **404**. After use, the zirconium oxide **404** will contain bound phosphate, fluoride and other anions, which can be replaced with hydroxide anions through the recharging process described herein. The direction of flow of dialysate through the sorbent cartridge is shown by the arrow in FIG. 4. The recharging solutions can also flow through the reusable sorbent modules in an opposite direction to improve the efficiency of the recharging process.

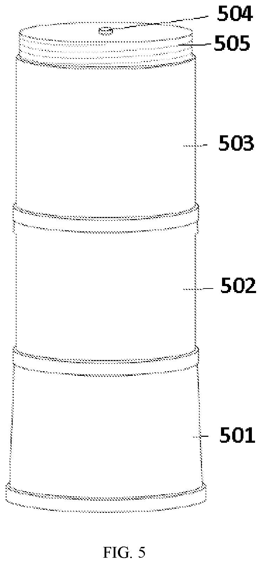

FIG. 5 illustrates another non-limiting example of a modular sorbent cartridge that can be used in the recharging process described herein. The modular sorbent cartridge can be separated into discrete modules including a first module **501**, a second module **502**, and a third module **503** connected together to form a sorbent cartridge. The first module **501** can contain activated carbon, urease, and alumina; the second module **502** can contain zirconium phosphate; and the third module **503** can contain zirconium oxide. One of skill in the art will understand that the modular sorbent cartridge illustrated in FIG. 5 is for illustrative purposes only, and modifications to the sorbent cartridge can be made within the scope of the invention. Alternatively, the sorbent modules can be independent with fluid lines connecting each of the sorbent modules for dialysis. During dialysis, dialysate can enter the sorbent cartridge through the bottom of first module **501**, travel through modules **501**, **502**, and **503**, and exit through fluid outlet **504**. The fluid outlet **504** can connect to the rest of the dialysate flow path. Threaded portion **505** on module **503** can be used in connecting modules to each other, to the dialysate flow path, or to the recharger as described herein. The threaded portion **505** can be included on any of the sorbent modules. Other connection types suitable for secured fluid connection in dialysis known in the art is contemplated by the invention. For example, fluid lines can be clamped directly onto fluid outlet **504**. After dialysis, a user can disconnect the sorbent modules for disposal of single use modules and for recharging of the reusable modules.

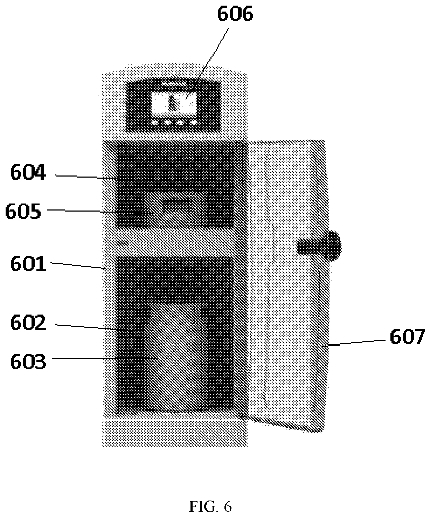

FIG. 6 illustrates a non-limiting embodiment of a recharger **601** configured for recharging zirconium phosphate and zirconium oxide modules. The recharger **601** can have a zirconium phosphate receiving compartment **602** configured to hold a zirconium phosphate sorbent module **603**. The recharger **601** can also have a zirconium oxide receiving compartment **604** configured to hold a zirconium oxide sorbent module **605**. Fluid connectors (not shown in FIG. 6) can provide fluid connection to the fluid sources described herein. One or more fluid sources can be housed within the recharger **601** or outside of the recharger **601** with fluid connectors connecting the fluid sources to the recharging flow paths. Fluid sources placed external to the recharger **601** can result in a compact recharger **601**. The recharger **601** can have a door **607** which can prevent access to the reusable modules during operation. The recharger **601** can also have a user interface **606**. The user interface **606** can start or control the recharging process by the user. Further, the user interface **606** can provide the status of the recharging process to the user such as the times to completion for each recharging step in FIG. 3. User interface **606** can also provide alert messages if any problems are detected during recharging, such as leaks, occlusions, pump failures, or mismatched chemicals. Rechargers with any number of receiving compartments for recharging any number or combination of zirconium oxide and/or zirconium phosphate sorbent modules can be constructed. For example, a recharger with two zirconium phosphate receiving compartments and two zirconium oxide receiving compartments can be similarly constructed. The rechargers can have 1, 2, 3, 4, 5, 6, or more receiving compartments, each capable of receiving zirconium oxide or zirconium phosphate sorbent modules.

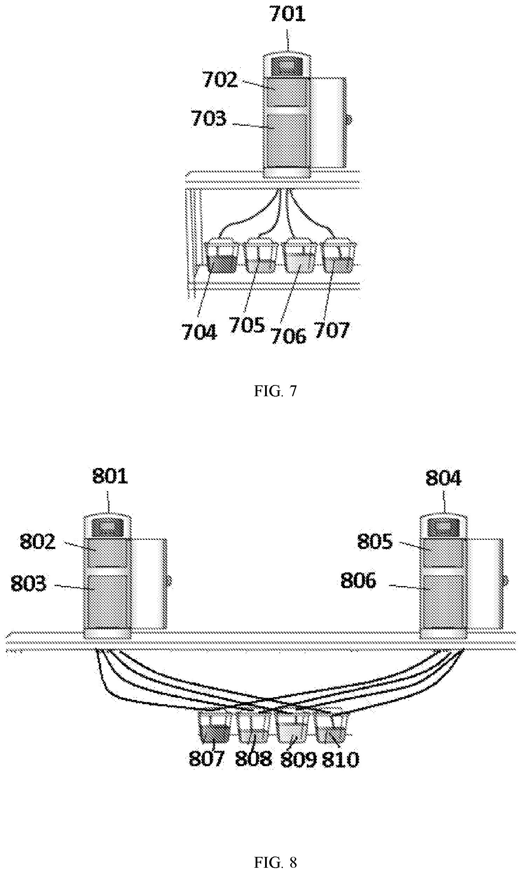

FIG. 7 illustrates a non-limiting embodiment of a recharger set up for recharging zirconium oxide and zirconium phosphate. The recharger **701** can have a zirconium phosphate receiving compartment **702** and a zirconium oxide receiving compartment **703**. External fluid sources such as water source **704**, brine source **705**, disinfectant source **706**, and base source **707** can be fluidly connected to the recharger **701.** The pumps, valves, fluid lines, and other components described in FIG.'s 1-2 can be fluidly connected to the fluid sources inside recharger **701**.

The rechargers can be used in any setting, including a clinic, at home, or in a mobile setting. In any setting, the rechargers can use a water tank or any other source of potable or deionized water. For use in a mobile setting, vans or trucks can carry the rechargers, the disinfectant source, the brine solution, the base solution, and optionally the water, to a location for recharging. For at home use, the brine solution, disinfectant solution, base solution, and optionally the water, may be prepackaged and shipped to a patient. The patient can connect each of the sources to the recharger to allow recharging and reuse of the sorbent modules in dialysis. As described, the rechargers can provide for inline mixing of chemicals, reducing the amount of chemicals required to be moved for use in a mobile setting. Inline mixing of chemicals allows for a smaller amount of concentrated solutions to be moved to a location in a mobile or at home setting, and water from a local water source, such as municipal drinking water, can be used to dilute the disinfectant, base, and/or brine inline. Alternatively, a deionized or purified water source can be provided in a mobile setting. Effluent from the sorbent modules can be collected and neutralized inline for immediate disposal in any drain, or can be collected for later neutralization and disposal offline. The ability to neutralize and dispose of the combined effluents in a drain allow for easier use in an at home or mobile setting, without the need for large waste reservoirs and further treatment.

In FIG. 8, multiple rechargers can be connected together to share fluid sources and to decrease space requirements and costs. A first recharger **801** having a zirconium phosphate receiving compartment **803** and zirconium oxide receiving compartment **802** can be fluidly connected to water source **807**, brine source **808**, disinfectant source **809**, and base source **810**. A second recharger **804** can also be fluidly connected to water source **807**, brine source **808**, disinfectant source **809**, and base source **810**. Any number of rechargers can be connected to a common set of fluid sources, including 2, 3, 4, 5, 6 or more rechargers each fluidly connected to a single set of fluid sources and a single set of waste reservoirs. Connecting multiple rechargers to a single set of fluid sources saves space and materials and simplified recharging multiple sets of reusable modules in a clinic or hospital setting. Each of the connected rechargers can have separate heaters for heating the brine and/or disinfectant solutions, or centralized heaters can be included, with centralized heating of the shared solutions.

One skilled in the art will understand that various combinations and/or modifications and variations can be made in the described systems and methods depending upon the specific needs for operation.

## Claims

1. A recharging flow path, comprising:
a zirconium phosphate recharging flow path (101) and a zirconium oxide recharging flow path (102);
the zirconium phosphate recharging flow path comprising:
i) a water source (105), a disinfectant source (107), and a brine source (106);
ii) a zirconium phosphate module inlet and a zirconium phosphate module outlet; wherein the zirconium phosphate module inlet and the zirconium phosphate module outlet are fluidly connectable to a sorbent module containing zirconium phosphate;
iii) at least one zirconium phosphate pump (109, 110) for conveying fluid from the water source, the disinfectant source, and the brine source to the zirconium phosphate module inlet; and
iv) a zirconium phosphate effluent line (139) in fluid connection to the zirconium phosphate module outlet;
the zirconium oxide recharging flow path comprising:
i) the water source, the disinfectant source, and a base source (108);
ii) a zirconium oxide module inlet and a zirconium oxide module outlet;
wherein the zirconium oxide module inlet and the zirconium oxide module outlet are fluidly connectable to a sorbent module containing zirconium oxide;
iii) at least one zirconium oxide pump (126, 127) for conveying fluid from the water source, the disinfectant source, and the base source to the zirconium oxide module inlet; and
iv) a zirconium oxide effluent line (138) in fluid connection to the zirconium oxide module outlet;
v) wherein the zirconium phosphate effluent line is fluidly connected to the zirconium oxide effluent line at an effluent line junction (140);
and further comprising a drain line (145) fluidly connected from the effluent line junction to a common waste reservoir.

2. The recharging flow path of claim 1, wherein either or both of the zirconium phosphate recharging flow path and the zirconium oxide recharging flow path comprise at least two pumps.

3. The recharging flow path of claim 1 or 2, further comprising a static mixer (146) downstream of the effluent line junction.

4. The recharging flow path of any preceding claim, further comprising a heater (119) positioned in the zirconium phosphate recharging flow path upstream of the zirconium phosphate module inlet.

5. The recharging flow path of claim 4, further comprising a heat exchanger (120); wherein the heat exchanger comprises at least a first chamber and a second chamber; wherein the first chamber is positioned in the zirconium phosphate recharging flow path upstream of the heater; and wherein the second chamber is positioned in the zirconium phosphate effluent line;
and preferably wherein the zirconium phosphate recharging flow path further comprises a rinse loop; wherein the rinse loop (151) is fluidly connected to a first valve (113) positioned in the zirconium phosphate recharging flow path upstream of the heater and heat exchanger; and the rinse loop is fluidly connected to a second valve (116) positioned in the zirconium phosphate recharging flow path downstream of the heater and heat exchanger and upstream of the zirconium phosphate module inlet.

6. The recharging flow path of any preceding claim, further comprising a heater positioned in the zirconium oxide recharging flow path upstream of the zirconium oxide module inlet;
preferably further comprising a heat exchanger; wherein the heat exchanger comprises at least a first chamber and a second chamber; wherein the first chamber is positioned in the zirconium oxide recharging flow path upstream of the heater; and wherein the second chamber is positioned in the zirconium oxide effluent line.

7. The recharging flow path of claim 6, wherein the zirconium oxide recharging flow path further comprises a rinse loop; wherein the rinse loop is fluidly connected to a first valve positioned in the zirconium oxide recharging flow path upstream of the heater and heat exchanger; and the rinse loop is fluidly connected to a second valve positioned in the zirconium oxide recharging flow path downstream of the heater and heat exchanger and upstream of the zirconium oxide module inlet.

8. The recharging flow path of any preceding claim, further comprising either or both of a zirconium phosphate bypass line (152) and a zirconium oxide bypass line (137); wherein the zirconium phosphate bypass line fluidly connects the zirconium phosphate recharging flow path at a position upstream of the zirconium phosphate module inlet to the zirconium phosphate effluent line; and wherein the zirconium oxide bypass line fluidly connects the zirconium oxide recharging flow path at a position upstream of the zirconium oxide module inlet to the zirconium oxide effluent line.

9. The recharging flow path of any preceding claim, further comprising a first sensor (141) positioned in the zirconium phosphate effluent line and a second sensor (144) positioned in the zirconium oxide effluent line.

10. The recharging flow path of any preceding claim, further comprising a first sensor (121) in the zirconium phosphate recharging flow path and a second sensor (133) in the zirconium oxide recharging flow path; preferably further comprising:
a first zirconium phosphate pump and a second zirconium phosphate pump; wherein the water source is fluidly connected to the zirconium phosphate recharging flow path through a first valve (129);
wherein either or both of the disinfectant source and brine source is fluidly connected to the zirconium phosphate recharging flow path through a second valve (212);
the first zirconium phosphate pump positioned in the zirconium phosphate recharging flow path downstream of the first valve and upstream of a static mixer; and the second zirconium phosphate pump positioned in the zirconium phosphate recharging flow path downstream of the second valve and upstream of the static mixer; preferably still wherein the first sensor is positioned downstream of the static mixer.

11. The recharging flow path of claim 10, further comprising:
a first zirconium oxide pump and a second zirconium oxide pump; wherein the water source is fluidly connected to the zirconium oxide recharging flow path through a first valve;
wherein the disinfectant source and the base source are fluidly connected to the zirconium oxide recharging flow path through a second valve (234);
wherein the first zirconium oxide pump is positioned in the zirconium oxide recharging flow path downstream of the first valve and upstream of a static mixer;
and the second zirconium oxide pump is positioned in the zirconium oxide recharging flow path downstream of the second valve and upstream of the static mixer;
preferably wherein the second sensor is positioned downstream of the static mixer.

12. The recharging flow path of any preceding claim, wherein at least one of the water source, disinfectant source, brine source, and base source is external to a recharger housing; preferably wherein at least one of the water source, disinfectant source, brine source, and base source are fluidly connected to a second recharging flow path.

13. The recharging flow path of any preceding claim, further comprising at least one conductivity sensor (219) positioned in the zirconium phosphate recharging flow path upstream of the zirconium phosphate module inlet, and at least a second conductivity sensor (239) positioned in the zirconium oxide recharging flow path upstream of the zirconium oxide module inlet.

14. The recharging flow path of any preceding claim, further comprising at least one of a pressure sensor (220) and flow sensor (222) fluidly positioned in the zirconium phosphate recharging flow path, and at least one of a pressure sensor (240) and flow sensor (241) positioned in the zirconium oxide recharging flow path; and/or wherein the disinfectant source is a peracetic acid solution having a concentration in a range between 0.5 % and 2% of peracetic acid in water.

15. The recharging flow path of any preceding claim, wherein the base source is a sodium hydroxide solution having a concentration greater than 2% of sodium hydroxide in water.

## Patentansprüche

1. Wiederbefüllungsströmungsweg, umfassend:
einen Zirkoniumphosphat-Wiederbefüllungsströmungsweg (101) und einen Zirkoniumoxid-Wiederbefüllungsströmungsweg (102);
der Zirkoniumphosphat-Wiederbefüllungsströmungsweg Folgendes umfassend:
i) eine Wasserquelle (105), Desinfektionsmittelquelle (107)) und eine Salzlösungsquelle (106);
ii) einen Zirkoniumphosphat-Moduleinlass und einen Zirkoniumphosphat-Modulauslass; wobei der Zirkoniumphosphat-Moduleinlass und der Zirkoniumphosphat-Modulauslass fluidtechnisch mit einem Zirkoniumphosphat enthaltendem Sorptionsmittel-Modul verbindbar sind;
iii) mindestens eine Zirkoniumphosphat-Pumpe (109, 110) zum Befördern von Fluid von der Wasserquelle, der Desinfektionsmittelquelle und der Salzlösungsquelle zu dem Zirkoniumphosphat-Moduleinlass; und
iv) eine mit dem Zirkoniumphosphat-Modulauslass in Fluidverbindung stehende Zirkoniumphosphat-Ausflussleitung (139);
der Zirkoniumoxid-Wiederbefüllungsströmungsweg Folgendes umfassend:
i) die Wasserquelle, die Desinfektionsmittelquelle und eine Basenquelle (108);
ii) einen Zirkoniumoxid-Moduleinlass und einen Zirkoniumoxid-Modulauslass; wobei der Zirkoniumoxid-Moduleinlass und der Zirkoniumoxid-Modulauslass fluidtechnisch mit einem Zirkoniumoxid enthaltenden Sorptionsmittel-Modul verbindbar sind;
iii) mindestens eine Zirkoniumoxid-Pumpe (126, 127) zum Befördern von Fluid von der Wasserquelle, der Desinfektionsmittelquelle und der Basenquelle zu dem Zirkoniumoxid-Moduleinlass; und
iv) eine mit dem Zirkoniumoxid-Modulauslass in Fluidverbindung stehende Zirkoniumoxid-Ausflussleitung (138);
v) wobei die Zirkoniumphosphat-Ausflussleitung bei einem Ausflussleitungsverbindungspunkt (140) fluidtechnisch mit der Zirkoniumoxid-Ausflussleitung verbunden ist; und ferner umfassend eine Ablaufleitung (145), die von dem
Ausflussleitungsverbindungspunkt aus fluidtechnisch mit einem gemeinsamen Abfallbehälter verbunden ist.

2. Wiederbefüllungsströmungsweg nach Anspruch 1, wobei entweder der Zirkoniumphosphat-Wiederbefüllungsströmungsweg oder der Zirkoniumoxid-Wiederbefüllungsströmungsweg oder beide mindestens zwei Pumpen umfassen.

3. Wiederbefüllungsflussweg nach Anspruch 1 oder 2, ferner umfassend einen statischen Mischer (146) stromabwärts von dem Abflussleitungsverbindungspunkt.

4. Wiederbefüllungsströmungsweg nach einem der vorhergehenden Ansprüche, ferner umfassend eine in dem Zirkoniumphosphat-Wiederbefüllungsströmungsweg stromaufwärts des Zirkoniumphosphat-Moduleinlasses angeordnete Heizung (119).

5. Wiederbefüllungsströmungsweg nach Anspruch 4, ferner umfassend einen Wärmetauscher (120); wobei der Wärmetauscher mindestens eine erste Kammer und eine zweite Kammer umfasst; wobei die erste Kammer in dem Zirkoniumphosphat-Wiederbefüllungsströmungsweg stromaufwärts der Heizung angeordnet ist; und wobei die zweite Kammer in der Zirkoniumphosphat-Ausflussleitung angeordnet ist;
und wobei ferner vorzugsweise der Zirkoniumphosphat-Wiederbefüllungsströmungsweg eine Spülschleife umfasst; wobei die Spülschleife (151) fluidtechnisch mit einem ersten in dem Zirkoniumphosphat-Wiederbefüllungsströmungsweg stromaufwärts der Heizung und des Wärmetauschers angeordneten Ventil (113) verbunden ist; und die Spülschleife fluidtechnisch mit einem zweiten in dem Zirkoniumphosphat-Wiederbefüllungsströmungsweg stromabwärts der Heizung und des Wärmetauschers und stromaufwärts des Zirkoniumphosphat-Moduleinlasses angeordneten Ventil (116) verbunden ist.

6. Wiederbefüllungsströmungsweg nach einem der vorhergehenden Ansprüche, ferner umfassend eine in dem Zirkoniumoxid-Wiederbefüllungsströmungsweg stromaufwärts des Zirkoniumoxid-Moduleinlasses angeordnete Heizung;
vorzugsweise ferner umfassend einen Wärmetauscher; wobei der Wärmetauscher mindestens eine erste Kammer und eine zweite Kammer umfasst; wobei die erste Kammer in dem Zirkoniumoxid-Wiederbefüllungsströmungsweg stromaufwärts der Heizung angeordnet ist; und wobei die zweite Kammer in der Zirkoniumoxid-Ausflussleitung angeordnet ist.

7. Wiederbefüllungsströmungsweg nach Anspruch 6, wobei der Zirkoniumoxid-Wiederbefüllungsströmungsweg ferner eine Spülschleife umfasst; wobei die Spülschleife fluidtechnisch mit einem ersten in dem Zirkoniumoxid-Wiederbefüllungsströmungsweg stromaufwärts der Heizung und des Wärmetauschers angeordneten Ventil verbunden ist; und die Spülschleife fluidtechnisch mit einem zweiten in dem Zirkoniumoxid-Wiederbefüllungsströmungsweg stromabwärts der Heizung und des Wärmetauschers und stromaufwärts des Zirkoniumoxid-Moduleinlasses angeordneten Ventil verbunden ist.

8. Wiederbefüllungsströmungsweg nach einem der vorhergehenden Ansprüche, ferner umfassend entweder eine Zirkoniumphosphat-Umführungsleitung (152) oder eine Zirkoniumoxid-Umführungsleitung (137) oder beides; wobei die Zirkoniumphosphat-Umführungsleitung den Zirkoniumphosphat-Wiederbefüllungsströmungsweg an einer Stelle stromaufwärts des Zirkoniumphosphat-Moduleinlasses fluidtechnisch mit der Zirkoniumphosphat-Ausflussleitung verbindet; und wobei die Zirkoniumoxid-Umführungsleitung den Zirkoniumoxid-Wiederbefüllungsströmungsweg an einer Stelle stromaufwärts des Zirkoniumoxid-Moduleinlasses fluidtechnisch mit der Zirkoniumoxid-Ausflussleitung verbindet.

9. Wiederbefüllungsströmungsweg nach einem der vorhergehenden Ansprüche, ferner umfassend einen ersten in der Zirkoniumphosphat-Ausflussleitung angeordneten Sensor (141) und einen zweiten in der Zirkoniumoxid-Ausflussleitung angeordneten Sensor (144).

10. Wiederbefüllungsströmungsweg nach einem der vorhergehenden Ansprüche, ferner umfassend einen ersten Sensor (121) in dem Zirkoniumphosphat-Wiederbefüllungsströmungsweg und einen zweiten Sensor (133) in dem Zirkoniumoxid-Wiederbefüllungsströmungsweg; vorzugsweise ferner Folgendes umfassend:
eine erste Zirkoniumphosphat-Pumpe und eine zweite Zirkoniumphosphat-Pumpe; wobei die Wasserquelle durch ein erstes Ventil (129) fluidtechnisch mit dem Zirkoniumphosphat-Wiederbefüllungsströmungsweg verbunden ist;
wobei entweder die Desinfektionsmittelquelle oder die Salzlösungsquelle oder beide durch ein zweites Ventil (212) fluidtechnisch mit dem Zirkoniumphosphat-Wiederbefüllungsströmungsweg verbunden sind;
wobei die erste Zirkoniumphosphat-Pumpe in dem Zirkoniumphosphat-Wiederbefüllungsströmungsweg stromabwärts des ersten Ventils und stromaufwärts eines statischen Mischers angeordnet ist; und die zweite Zirkoniumphosphat-Pumpe in dem Zirkoniumphosphat-Wiederbefüllungsströmungsweg stromabwärts des zweiten Ventils und stromaufwärts des statischen Mischers angeordnet ist; wobei vorzugsweise ferner der erste Sensor stromabwärts des statischen Mischers angeordnet ist.

11. Wiederbefüllungsströmungsweg nach Anspruch 10, ferner Folgendes umfassend:
eine erste Zirkoniumoxid-Pumpe und eine zweite Zirkoniumoxid-Pumpe; wobei die Wasserquelle durch ein erstes Ventil fluidtechnisch mit dem Zirkoniumoxid-Wiederbefüllungsströmungsweg verbunden ist;
wobei die Desinfektionsmittelquelle und die Basenquelle durch ein zweites Ventil (234) fluidtechnisch mit dem Zirkoniumoxid-Wiederbefüllungsströmungsweg verbunden sind;
wobei die erste Zirkoniumoxid-Pumpe in dem Zirkoniumoxid-Wiederbefüllungsströmungsweg stromabwärts des ersten Ventils und stromaufwärts eines statischen Mischers angeordnet ist;
und wobei die zweite Zirkoniumoxid-Pumpe in dem Zirkoniumoxid-Wiederbefüllungsströmungsweg stromabwärts des zweiten Ventils und stromaufwärts des statischen Mischers angeordnet ist;
wobei vorzugsweise der zweite Sensor stromabwärts des statischen Mischers angeordnet ist.

12. Wiederbefüllungsströmungsweg nach einem der vorhergehenden Ansprüche, wobei sich mindestens eines von der Wasserquelle, Desinfektionsmittelquelle, Salzlösungsquelle und Basenquelle außerhalb eines Wiederbefüllungsgehäuses befindet; wobei vorzugsweise mindestens eines von der Wasserquelle, Desinfektionsmittelquelle, Salzlösungsquelle und Basenquelle fluidtechnisch mit einem zweiten Wiederbefüllungsströmungsweg verbunden sind.

13. Wiederbefüllungsströmungsweg nach einem der vorhergehenden Ansprüche, ferner umfassend mindestens einen in dem Zirkoniumphosphat-Wiederbefüllungsströmungsweg stromaufwärts des Zirkoniumphosphat-Moduleinlasses angeordneten Leitfähigkeitssensor (219) und mindestens einen zweiten in dem Zirkoniumoxid-Wiederbefüllungsströmungsweg stromaufwärts des Zirkoniumoxid-Moduleinlasses angeordneten Leitfähigkeitssensor (239).

14. Wiederbefüllungsströmungsweg nach einem der vorhergehenden Ansprüche, ferner umfassend mindestens eines von einem Drucksensor (220) und einem Strömungssensor (222), die fluidtechnisch in dem Zirkoniumphosphat-Wiederbefüllungsströmungsweg angeordnet sind, mindestens eines von einen Drucksensor (240) und einem Strömungssensor (241), die in dem Zirkoniumoxid-Wiederbefüllungsströmungsweg angeordnet sind; und/oder wobei die Desinfektionsmittelquelle eine Peressigsäurelösung ist, die eine Konzentration in einem Bereich zwischen 0,5 % und 2 % Peressigsäure in Wasser aufweist.

15. Wiederbefüllungsströmungsweg nach einem der vorhergehenden Ansprüche, wobei die Basenquelle eine Natriumhydroxidlösung ist, die eine Konzentration von mehr als 2 % Natriumhydroxid in Wasser aufweist.

## Revendications

1. Trajet d'écoulement de recharge, comprenant :
un trajet d'écoulement de recharge en phosphate de zirconium (101) et un trajet d'écoulement de recharge en oxyde de zirconium (102) ;
le trajet d'écoulement de recharge en phosphate de zirconium comprenant :
i) une source d'eau (105), une source de désinfectant (107) et une source de saumure (106) ;
ii) une entrée de module de phosphate de zirconium et une sortie de module de phosphate de zirconium ; dans lequel l'entrée de module de phosphate de zirconium et la sortie de module de phosphate de zirconium peuvent être reliées de manière fluidique à un module de sorbant contenant du phosphate de zirconium ;
iii) au moins une pompe à phosphate de zirconium (109, 110) pour transporter le fluide depuis la source d'eau, la source de désinfectant et la source de saumure vers l'entrée du module de phosphate de zirconium ; et
iv) une conduite d'effluent de phosphate de zirconium (139) en liaison fluidique avec la sortie du module de phosphate de zirconium ;
le trajet d'écoulement de recharge d'oxyde de zirconium comprenant :
i) la source d'eau, la source de désinfectant et une source de base (108) ;
ii) une entrée de module d'oxyde de zirconium et une sortie de module d'oxyde de zirconium ; dans lequel l'entrée de module d'oxyde de zirconium et la sortie de module d'oxyde de zirconium peuvent être reliées de manière fluidique à un module de sorbant contenant de l'oxyde de zirconium ;
iii) au moins une pompe à oxyde de zirconium (126, 127) pour transporter le fluide depuis la source d'eau, la source de désinfectant et la source de base vers l'entrée du module d'oxyde de zirconium ; et
iv) une conduite d'effluent d'oxyde de zirconium (138) en liaison fluidique avec la sortie de module d'oxyde de zirconium ;
v) dans lequel la conduite d'effluent de phosphate de zirconium est reliée de manière fluidique à la conduite d'effluent d'oxyde de zirconium au niveau d'une jonction de conduite d'effluent (140) ;
et comprenant en outre une conduite de drainage (145) reliée de manière fluidique de la jonction de conduite d'effluent à un réservoir de déchets commun.

2. Trajet d'écoulement de recharge selon la revendication 1, dans lequel le trajet d'écoulement de recharge en phosphate de zirconium et/ou le trajet d'écoulement de recharge en oxyde de zirconium comprennent au moins deux pompes.

3. Trajet d'écoulement de recharge selon la revendication 1 ou 2, comprenant en outre un mélangeur statique (146) en aval de la jonction de la conduite d'effluent.

4. Trajet d'écoulement de recharge selon l'une quelconque des revendications précédentes, comprenant en outre un dispositif de chauffage (119) positionné dans le trajet d'écoulement de recharge en phosphate de zirconium en amont de l'entrée de module de phosphate de zirconium.

5. Trajet d'écoulement de recharge selon la revendication 4, comprenant en outre un échangeur de chaleur (120) ; dans lequel l'échangeur de chaleur comprend au moins une première chambre et une seconde chambre ; dans lequel la première chambre est positionnée dans le trajet d'écoulement de recharge en phosphate de zirconium en amont du dispositif de chauffage ; et dans lequel la seconde chambre est positionnée dans la conduite d'effluent de phosphate de zirconium ;
et de préférence dans lequel le trajet d'écoulement de recharge en phosphate de zirconium comprend en outre une boucle de rinçage ; dans lequel la boucle de rinçage (151) est reliée de manière fluidique à une première vanne (113) positionnée dans le trajet d'écoulement de recharge en phosphate de zirconium en amont du dispositif de chauffage et de l'échangeur de chaleur ; et la boucle de rinçage est reliée de manière fluidique à une seconde vanne (116) positionnée dans le trajet d'écoulement de recharge en phosphate de zirconium en aval du dispositif de chauffage et de l'échangeur de chaleur et en amont de l'entrée de module de phosphate de zirconium.

6. Trajet d'écoulement de recharge selon l'une quelconque des revendications précédentes, comprenant en outre un dispositif de chauffage positionné dans le trajet d'écoulement de recharge en oxyde de zirconium en amont de l'entrée de module d'oxyde de zirconium ;
comprenant en outre de préférence un échangeur de chaleur ; dans lequel l'échangeur de chaleur comprend au moins une première chambre et une seconde chambre ; dans lequel la première chambre est positionnée dans le trajet d'écoulement de recharge en oxyde de zirconium en amont du dispositif de chauffage ; et dans lequel la seconde chambre est positionnée dans la conduite d'effluent d'oxyde de zirconium.

7. Trajet d'écoulement de recharge selon la revendication 6, dans lequel le chemin d'écoulement de recharge en oxyde de zirconium comprend en outre une boucle de rinçage ; dans lequel la boucle de rinçage est reliée de manière fluidique à une première vanne positionnée dans le trajet d'écoulement de recharge en oxyde de zirconium en amont du dispositif de chauffage et de l'échangeur de chaleur ; et la boucle de rinçage est reliée de manière fluidique à une seconde vanne positionnée dans le trajet d'écoulement de recharge en oxyde de zirconium en aval du dispositif de chauffage et de l'échangeur de chaleur et en amont de l'entrée de module d'oxyde de zirconium.

8. Trajet d'écoulement de recharge selon l'une quelconque des revendications précédentes, comprenant en outre l'une ou les deux d'une conduite de dérivation de phosphate de zirconium (152) et d'une conduite de dérivation d'oxyde de zirconium (137) ; dans lequel la conduite de dérivation de phosphate de zirconium relie de manière fluidique le trajet d'écoulement de recharge en phosphate de zirconium à une position en amont de l'entrée de module de phosphate de zirconium à la conduite d'effluent de phosphate de zirconium ; et dans lequel la conduite de dérivation d'oxyde de zirconium relie de manière fluidique le trajet d'écoulement de recharge en oxyde de zirconium à une position en amont de l'entrée du module d'oxyde de zirconium à la conduite d'effluent d'oxyde de zirconium.

9. Trajet d'écoulement de recharge selon l'une quelconque des revendications précédentes, comprenant en outre un premier capteur (141) positionné dans la conduite d'effluent de phosphate de zirconium et un second capteur (144) positionné dans la conduite d'effluent d'oxyde de zirconium.

10. Trajet d'écoulement de recharge selon l'une quelconque des revendications précédentes, comprenant en outre un premier capteur (121) dans le trajet d'écoulement de recharge en phosphate de zirconium et un second capteur (133) dans le trajet d'écoulement de recharge en oxyde de zirconium ; comprenant en outre de préférence :
une première pompe à phosphate de zirconium et une seconde pompe à phosphate de zirconium ; dans lequel la source d'eau est reliée de manière fluidique au trajet d'écoulement de recharge de phosphate de zirconium à travers une première vanne (129) ;
dans lequel l'une ou les deux de la source de désinfectant et de la source de saumure est reliée de manière fluidique au trajet d'écoulement de recharge en phosphate de zirconium à travers une seconde vanne (212) ;
la première pompe à phosphate de zirconium positionnée dans le trajet d'écoulement de recharge en phosphate de zirconium en aval de la première vanne et en amont d'un mélangeur statique ; et la seconde pompe à phosphate de zirconium positionnée dans le trajet d'écoulement de recharge en phosphate de zirconium en aval de la seconde vanne et en amont du mélangeur statique ; de préférence encore dans lequel le premier capteur est positionné en aval du mélangeur statique.

11. Trajet d'écoulement de recharge selon la revendication 10, comprenant en outre :
une première pompe à oxyde de zirconium et une seconde pompe à oxyde de zirconium ; dans lequel la source d'eau est reliée de manière fluidique au trajet d'écoulement de recharge en oxyde de zirconium à travers une première vanne ;
dans lequel la source de désinfectant et la source de base sont reliées de manière fluidique au trajet d'écoulement de recharge en oxyde de zirconium à travers une seconde vanne (234) ;
dans lequel la première pompe à oxyde de zirconium est positionnée dans le trajet d'écoulement de recharge en oxyde de zirconium en aval de la première vanne et en amont d'un mélangeur statique ;
et la seconde pompe à oxyde de zirconium est positionnée dans le trajet d'écoulement de recharge en oxyde de zirconium en aval de la seconde vanne et en amont du mélangeur statique ;
de préférence dans lequel le second capteur est positionné en aval du mélangeur statique.

12. Trajet d'écoulement de recharge selon l'une quelconque des revendications précédentes, dans lequel la source d'eau, et/ou la source de désinfectant, et/ou la source de saumure et/ou la source de base est externe à un boîtier de chargeur ; de préférence dans lequel la source d'eau, et/ou la source de désinfectant, et/ou la source de saumure et/ou la source de base sont reliées de manière fluidique à un second trajet d'écoulement de recharge.

13. Trajet d'écoulement de recharge selon l'une quelconque des revendications précédentes, comprenant en outre au moins un capteur de conductivité (219) positionné dans le trajet d'écoulement de recharge en phosphate de zirconium en amont de l'entrée de module en phosphate de zirconium, et au moins un second capteur de conductivité (239) positionné dans le trajet d'écoulement de recharge en oxyde de zirconium en amont de l'entrée de module d'oxyde de zirconium.

14. Trajet d'écoulement de recharge selon l'une quelconque des revendications précédentes, comprenant en outre un capteur de pression (220) et/ou un capteur d'écoulement (222) positionnés de manière fluidique dans le trajet d'écoulement de recharge en phosphate de zirconium, et un capteur de pression (240) et/ou un capteur d'écoulement (241) positionné dans le trajet d'écoulement de recharge en oxyde de zirconium ; et/ou la source de désinfectant étant une solution d'acide peracétique ayant une concentration dans une plage comprise entre 0,5 % et 2 % d'acide peracétique dans l'eau.

15. Trajet d'écoulement de recharge selon l'une quelconque des revendications précédentes, dans lequel la source de base est une solution d'hydroxyde de sodium ayant une concentration supérieure à 2 % d'hydroxyde de sodium dans l'eau.
